# EUROPEAN PATENT SPECIFICATION

(11) **EP 2 748 163 B1**
(45) Date of publication and mention of the grant of the patent: **20.04.2016**
(21) Application number: 12727948.7
(22) Date of filing: 30.05.2012
(51) Int. Cl.: C07D 471/04

(54) **PROCESS FOR PREPARING SYNTHETIC INTERMEDIATES WHICH CAN BE USED FOR MANUFACTURING ANTIBIOTICS**
VERFAHREN ZUR HERSTELLUNG SYNTHETISCHE ZWISCHENPRODUKTE, DIE ZUR HERSTELLUNG VON ANTIBIOTIKA DIENEN
PROCÉDÉ POUR LA PRÉPARATION DES PRODUITS INTERMÉDIARES UTILES DANS LA FABRICATION D'ANTIBIOTIQUES

(30) Priority: 31.05.2011 WO PCT/IB2011/052387
(43) Date of publication of application: 02.07.2014
(73) Proprietor: Actelion Pharmaceuticals Ltd., 4123 Allschwil (CH)
(72) Inventor: ABELE, Stefan, CH-4123 Allschwil (CH); FUNEL, Jacques-Alexis, CH-4123 Allschwil (CH); SCHMIDT, Gunther, CH-4123 Allschwil (CH); SURIVET, Jean-Philippe, CH-4123 Allschwil (CH)
(74) Representative: Ruhlmann, Eric
(86) International application number: PCT/IB2012/052702
(87) International publication number: WO 2012/164498

(56) References cited:
- WO-A1-2008/152603
- WO-A1-2010/067332

## Description

The present invention relates to a process for preparing synthetic intermediates and the use of these intermediates for manufacturing antibiotics.

WO 2010/067332 discloses antibiotic compounds having the core structure (A) represented below 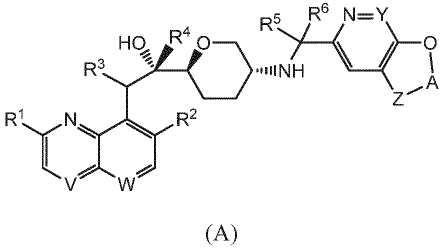 wherein R¹ represents alkoxy, R² represents H or F, each of R³, R⁴, R⁵ and R⁶ represents independently H or D, V represents CH and W represents CH or N or V represents N and W represents CH, Y represents CH or N, Z represents O, S or CH₂, and A represents CH₂, CH₂CH₂ or CD₂CD₂.

The preparation routes described for the compounds of formula (A) in WO 2010/067332, and notably for those wherein V represents CH and W represents N, R¹ represents methoxy, R² represents F and each of R³ and R⁴ represents H (hereafter referred to as "compounds of formula (A1)") which use as an intermediate the compound of formula I-4_{BOC} 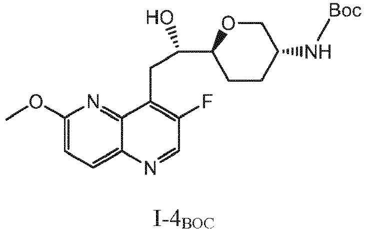 are however not most appropriate for large manufacturing. It is therefore desirable to achieve a cheaper, more efficient and more environment-friendly preparation of such compounds (e.g. avoiding use of heavy metals).

The Applicants have now found an improved manufacturing route to obtain the compounds of formula (A1), which uses the intermediates of formula I-1 below 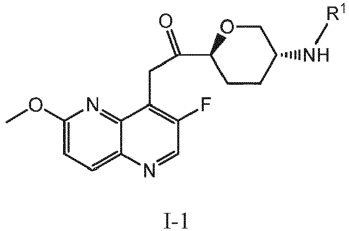 wherein R¹ represents hydrogen or a group -CO-R² wherein R² represents trifluoromethyl, *tert*-butoxy, benzyloxy or 4-methoxybenzyloxy.

WO 2008/152603 discloses the compounds of formula (B) 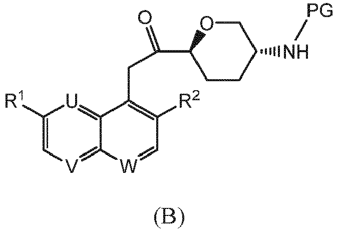 wherein
R¹ represents halogen or (C₁-C₄)alkoxy;
U and W each represent N, V represents CH and R² represents H or F, or U and V each represent CH, W represents N and R² represents H or F, U represents N, V represents CH, W represents CH or CR^{a} and R² represents H, or also, when W represents CH, may represent F;
R^{a} represents CH₂OH or [(C₁-C₄)alkoxy]carbonyl; and
PG is an amino protecting group such as Cbz or Boc.

Besides, WO 2010/067332 discloses the compounds of formula (C) 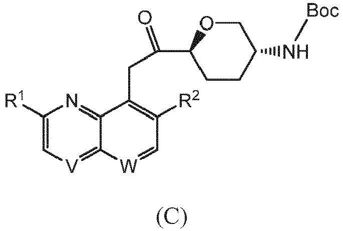 wherein
R¹ represents (C₁-C₄)alkoxy;
R² represents H or F; and
V represents CH and W represents CH or N or V represents N and W represents CH. These compounds of formula (C) have been described in general preparation routes for obtaining certain deuterated derivatives of compounds of formula (A).

After stereoselective reduction of the intermediate of formula I-1 according to the present invention, the protecting group -CO-R² can be removed by methods known to the skilled artisan and the synthesis of the desired compounds of formula (A1) can be easily achieved (for example using the methods disclosed in WO 2010/067332).

Various embodiments of the invention are presented hereafter:
1) The invention relates to the use of the compound of formula I-1_{BOC} 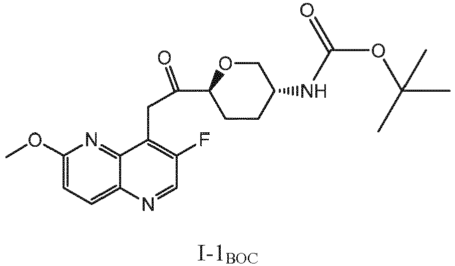 in a process for the stereoselective preparation of the compound of formula I-4_{BOC} 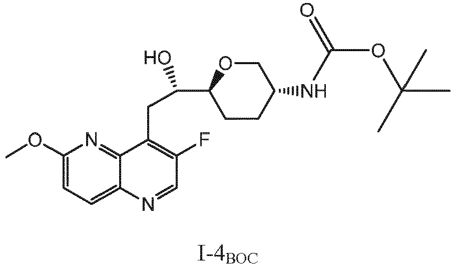 said process comprising the reaction, in a polar aprotic solvent or a polar aprotic mixture of solvents, of the compound of formula I-1_{BOC}
   a) either with DIBAH,
   b) or with triisopropoxyaluminium, dimethylaluminium chloride or diethylaluminium chloride in the presence of iPrOH.
2) According to one variant of the process of embodiment 1), the reaction will be performed with DIBAH.
3) Preferably, the reaction of the compound of formula I-1_{BOC} with DIBAH in the process according to embodiment 1) or 2) will be performed at a temperature of -30°C or below.
4) More preferably, the reaction of a compound of formula I-1_{BOC} with DIBAH in the process according to embodiment 1) or 2) will be performed at a temperature of -65°C or below (for example at a temperature of about -78°C).
5) In particular, the polar aprotic solvent of the process according to one of embodiments 2) to 4) will comprise THF (whereby such polar aprotic solvent or the polar aprotic mixture of solvents will notably be selected from THF and a mixture of THF and toluene and more particularly will consist exclusively of THF).
6) According to a preferred variant of the process according to one of embodiments 1) to 5), the reaction with DIBAH will be performed using from 1.5 to 10 equivalents of DIBAH per equivalent of compound of formula I-1_{BOC}, and notably from 2 to 6 equivalents of DIBAH per equivalent of compound of formula I-1_{BOC}.
7) In particular, according to the preferred variant of the process according to embodiment 6), the reaction with DIBAH will be performed using from 2 to 4 equivalents of DIBAH per equivalent of compound of formula I-1_{BOC} (and notably about 2.2 equivalents of DIBAH per equivalent of compound of formula I-1_{BOC}).
8) According to one particular method of performing the process according to embodiments 2) to 7), the appropriate quantity of DIBAH is added over a period of time to a solution of the appropriate quantity of compound of formula I-1_{BOC} in a polar aprotic solvent or a polar aprotic solvent mixture.
9) According to another particular method of performing the process according to embodiments 2) to 7), the appropriate quantity of compound of formula I-1_{BOC} is added over a period of time to a solution of the appropriate quantity of DIBAH in a polar aprotic solvent or a polar aprotic solvent mixture.
10) According to another variant of the process of embodiment 1), the reaction will be performed with triisopropoxyaluminium in the presence of iPrOH.
11) Preferably, a process according to embodiment 10) will be performed using about 2 equivalents of triisopropoxyaluminium per equivalent of compound of formula I-1_{BOC}.
12) Preferably, a process according to embodiment 10) or 11) will be performed at a temperature of at least 25°C (and more preferably at a temperature of at least 50°C, for example at a temperature of about 80°C).
13) According to yet another variant of the process of embodiment 1), the reaction will be performed with dimethylaluminium chloride or diethylaluminium chloride in the presence of iPrOH (and in particular with dimethylaluminium chloride in the presence of iPrOH).
14) Preferably, a process according to embodiment 13) will be performed using about 2 to 3 equivalents of dimethylaluminium chloride or diethylaluminium chloride per equivalent of compound of formula I-1_{BOC} (and in particular about 3 equivalents of dimethylaluminium chloride per equivalent of compound of formula I-1_{BOC}).
15) Preferably, a process according to embodiment 13) or 14) will be performed at a temperature of at least 20°C (and more preferably at a temperature of at least 30°C, for example at a temperature of about 40°C).
16) Preferably, a process according to one of embodiments 10) to 15) will be performed in the presence of at least 3 equivalents of iPrOH per equivalent of compound of formula I-1_{BOC}.
17) More preferably, a process according to one of embodiments 10) to 15) will be performed in the presence of at least 5 equivalents of iPrOH per equivalent of compound of formula I-1_{BOC}, particularly in the presence of at least 8 equivalents of iPrOH per equivalent of compound of formula I-1_{BOC} (for example in the presence of about 10 equivalents of iPrOH per equivalent of compound of formula I-1_{BOC}).
18) The invention furthermore relates to the use of the compound of formula I-1_{BOC} as defined in embodiment 1), in a process for the stereoselective preparation of the compound of formula I-5 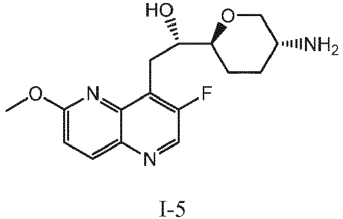 said process comprising:
   a) the reaction of the compound of formula I-1_{BOC}, in a polar aprotic solvent or a polar aprotic solvent mixture, either with DIBAH, or with triisopropoxyaluminium, dimethylaluminium chloride or diethylaluminium chloride in the presence of iPrOH, in particular according to the process of one of embodiments 1) to 17), affording the compound of formula I-4_{BOC} 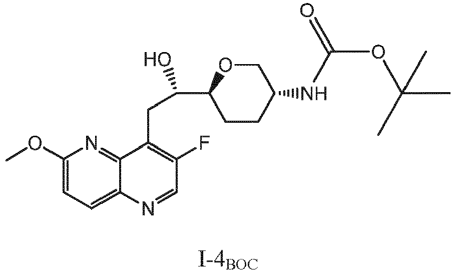 and
   b) the deprotection reaction, in acidic conditions, of the compound of formula I-4_{BOC}, affording the compound of formula I-5.
19) Preferably, the acidic conditions of the step b) of the process according to embodiment 18) will be provided by one of the following:
   ❖ reacting a solution of the compound of formula I-4_{BOC} with TFA;
   ❖ bubbling gaseous hydrochloric acid into a solution of the compound of formula I-4_{BOC}; or
   ❖ reacting the compound of formula I-4_{BOC}, or a solution thereof, with a solution of hydrochloric acid in dioxane, MeOH, EtOH, iPrOH or EA.
20) In particular, the solvent for the solution of the compound of formula I-4_{BOC} possibly used in step b) of the process according to embodiment 19) will be selected from the group consisting of DCM, EA, THF, 2-methyl-tetrahydrofurane, dioxane, toluene and iPrOH.
21) More preferably, the acidic conditions of the step b) of the process according to one of embodiments 18) to 20) will be provided by reacting the compound of formula I-4_{BOC}, or a solution thereof, with a solution of hydrochloric acid in iPrOH.
22) The invention further relates to a process for the stereoselective preparation of a compound of formula I-4 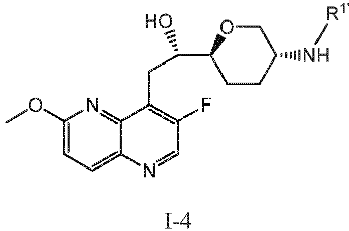 wherein R¹ represents hydrogen or a group -CO-R^{2'} wherein R^{2'} represents *tert*-butoxy, benzyloxy or 4-methoxybenzyloxy, said process comprising the reaction, in a polar aprotic solvent or a polar aprotic mixture of solvents, of a compound of formula I-1 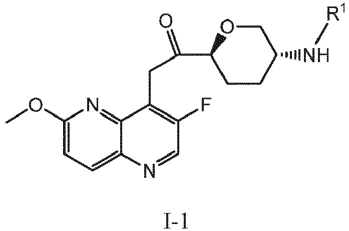 wherein R¹ represents hydrogen or a group -CO-R² wherein R² represents trifluoromethyl, *tert*-butoxy, benzyloxy or 4-methoxybenzyloxy
   a) either with DIBAH,
   b) or with triisopropoxyaluminium, dimethylaluminium chloride or diethylaluminium chloride in the presence of iPrOH.
23) According to one main variant of the process according to embodiment 40), the compound of formula I-1 will be such that R¹ represents hydrogen.
24) According to one sub-variant of embodiment 23), a process according to embodiment 41) will comprise the reaction of the compound of formula I-1 with DIBAH.
25) In a process according to embodiment 24), the reaction will preferably be performed using from 1.5 to 10 equivalents of DIBAH per equivalent of compound of formula I-1, and notably from 2 to 6 equivalents of DIBAH per equivalent of compound of formula I-1.
26) In a process according to embodiment 24), the reaction will more preferably be performed using from 2 to 4 equivalents of DIBAH per equivalent of compound of formula I-1 (and notably about 2.2 equivalents of DIBAH per equivalent of compound of formula I-1).
27) Preferably, the reaction of the compound of formula I-1 with DIBAH in the process according to one of embodiments 24) to 26) will be performed at a temperature of -30°C or below.
28) More preferably, the reaction of a compound of formula I-1 with DIBAH in the process according to one of embodiments 24) to 26) will be performed at a temperature of -65°C or below (for example at a temperature of about -78°C).
29) In a process according to one of embodiments 24) to 28), the polar aprotic solvent or a polar aprotic mixture of solvents will comprise THF (whereby such polar aprotic solvent or the polar aprotic mixture of solvents will notably be selected from THF and a mixture of THF and toluene and more particularly will consist exclusively of THF).
30) According to one particular method of performing the process according to embodiments 24) to 29), the appropriate quantity of DIBAH is added over a period of time to a solution of the appropriate quantity of compound of formula I-1 in a polar aprotic solvent or a polar aprotic solvent mixture.
31) According to another particular method of performing the process according to embodiments 24) to 29), the appropriate quantity of compound of formula I-1 is added over a period of time to a solution of the appropriate quantity of DIBAH in a polar aprotic solvent or a polar aprotic solvent mixture.
32) According to another sub-variant of embodiment 23), a process according to embodiment 23) will comprise the reaction of a compound of formula I-1 with triisopropoxyaluminium in the presence of iPrOH.
33) Preferably, a process according to embodiment 32) will be performed using about 2 equivalents of triisopropoxyaluminium per equivalent of compound of formula I-1.
34) Preferably, a process according to embodiment 32) or 33) will be performed at a temperature of at least 25°C (and more preferably at a temperature of at least 50°C, for example at a temperature of about 80°C).
35) According to yet another sub-variant of the process of embodiment 23), a process according to embodiment 23) will comprise the reaction of a compound of formula I-1 with dimethylaluminium chloride or diethylaluminium chloride in the presence of iPrOH (and in particular with dimethylaluminium chloride in the presence of iPrOH).
36) Preferably, a process according to embodiment 37) will be performed using about 2 to 3 equivalents of dimethylaluminium chloride or diethylaluminium chloride per equivalent of compound of formula I-1 (and in particular about 3 equivalents of dimethylaluminium chloride per equivalent of compound of formula I-1).
37) Preferably, a process according to embodiment 35) or 36) will be performed at a temperature of at least 20°C (and more preferably at a temperature of at least 30°C, for example at a temperature of about 40°C).
38) Preferably, a process according to one of embodiments 32) to 37) will be performed in the presence of at least 3 equivalents of iPrOH per equivalent of compound of formula 1-1.
39) More preferably, a process according to one of embodiments 32) to 37) will be performed in the presence of at least 5 equivalents of iPrOH per equivalent of compound of formula I-1, particularly in the presence of at least 8 equivalents of iPrOH per equivalent of compound of formula I-1 (for example in the presence of about 10 equivalents of iPrOH per equivalent of compound of formula I-1).
40) According to another main variant of the process according to embodiment 22), the compound of formula I-1 will be such that R¹ represents a group -CO-R².
41) According to one sub-variant of the process according to embodiment 40), the compound of formula I-1 will be such that R² represents trifluoromethyl and a compound of formula I-4 wherein R¹ represents hydrogen will be obtained.
42) According to a particular sub-embodiment of embodiment 41), a process according to embodiment 41) will comprise the reaction of the compound of formula I-1 with DIBAH.
43) In a process according to embodiment 42), the reaction will preferably be performed using from 1.5 to 10 equivalents of DIBAH per equivalent of compound of formula I-1, and notably from 2 to 6 equivalents of DIBAH per equivalent of compound of formula I-1.
44) In a process according to embodiment 42), the reaction will more preferably be performed using from 2 to 4 equivalents of DIBAH per equivalent of compound of formula I-1 (and notably about 2.2 equivalents of DIBAH per equivalent of compound of formula I-1).
45) Preferably, the reaction of the compound of formula I-1 with DIBAH in the process according to one of embodiments 42) to 44) will be performed at a temperature of -30°C or below.
46) More preferably, the reaction of a compound of formula I-1 with DIBAH in the process according to one of embodiments 42) to 44) will be performed at a temperature of -65°C or below (for example at a temperature of about -78°C).
47) In a process according to one of embodiments 42) to 46), the polar aprotic solvent or a polar aprotic mixture of solvents will comprise THF (whereby such polar aprotic solvent or the polar aprotic mixture of solvents will notably be selected from THF and a mixture of THF and toluene and more particularly will consist exclusively of THF).
48) According to one particular method of performing the process according to embodiments 42) to 47), the appropriate quantity of DIBAH is added over a period of time to a solution of the appropriate quantity of compound of formula I-1 in a polar aprotic solvent or a polar aprotic solvent mixture.
49) According to another particular method of performing the process according to embodiments 42) to 47), the appropriate quantity of compound of formula I-1 is added over a period of time to a solution of the appropriate quantity of DIBAH in a polar aprotic solvent or a polar aprotic solvent mixture.
50) According to another particular sub-embodiment of embodiment 41), a process according to embodiment 41) will comprise the reaction of the compound of formula I-1 with triisopropoxyaluminium in the presence of iPrOH.
51) Preferably, a process according to embodiment 50) will be performed using about 2 equivalents of triisopropoxyaluminium per equivalent of compound of formula I-1.
52) Preferably, a process according to embodiment 50) or 51) will be performed at a temperature of at least 25°C (and more preferably at a temperature of at least 50°C, for example at a temperature of about 80°C).
53) According to yet another particular sub-embodiment of embodiment 41), a process according to embodiment 59) will comprise the reaction of a compound of formula I-1 with dimethylaluminium chloride or diethylaluminium chloride in the presence of iPrOH (and in particular with dimethylaluminium chloride in the presence of iPrOH).
54) Preferably, a process according to embodiment 53) will be performed using about 2 to 3 equivalents of dimethylaluminium chloride or diethylaluminium chloride per equivalent of compound of formula I-1 (and in particular about 3 equivalents of dimethylaluminium chloride per equivalent of compound of formula I-1).
55) Preferably, a process according to embodiment 53) or 54) will be performed at a temperature of at least 20°C (and more preferably at a temperature of at least 30°C, for example at a temperature of about 40°C).
56) Preferably, a process according to one of embodiments 50) to 55) will be performed in the presence of at least 3 equivalents of iPrOH (and notably at least 5 equivalents of iPrOH) per equivalent of compound of formula I-1 (for example in the presence of about 10 equivalents of iPrOH per equivalent of compound of formula I-1).
57) According to another sub-variant of the process according to embodiment 41), the compound of formula I-1 will be such that R² represents *tert*-butoxy and a compound of formula I-4 wherein R^{1'} represents a group -CO-R^{2'} wherein R² represents *tert-*butoxy will be obtained.
58) According to a particular sub-embodiment of embodiment 57), a process according to embodiment 57) will comprise the reaction of the compound of formula I-1 thereof with DIBAH.
59) In a process according to embodiment 58), the reaction will preferably be performed using from 1.5 to 10 equivalents of DIBAH per equivalent of compound of formula I-1, and notably from 2 to 6 equivalents of DIBAH per equivalent of compound of formula I-1.
60) In a process according to embodiment 58), the reaction will more preferably be performed using from 2 to 4 equivalents of DIBAH per equivalent of compound of formula I-1 (and notably about 2.2 equivalents of DIBAH per equivalent of compound of formula I-1).
61) Preferably, the reaction of the compound of formula I-1 with DIBAH in the process according to one of embodiments 58) to 60) will be performed at a temperature of -30°C or below.
62) More preferably, the reaction of a compound of formula I-1 with DIBAH in the process according to one of embodiments 58) to 60) will be performed at a temperature of -65°C or below (for example at a temperature of about -78°C).
63) In a process according to one of embodiments 58) to 62), the polar aprotic solvent or a polar aprotic mixture of solvents will comprise THF (whereby such polar aprotic solvent or the polar aprotic mixture of solvents will notably be selected from THF and a mixture of THF and toluene and more particularly will consist exclusively of THF).
64) According to one particular method of performing the process according to embodiments 58) to 63), the appropriate quantity of DIBAH is added over a period of time to a solution of the appropriate quantity of compound of formula I-1 in a polar aprotic solvent or a polar aprotic solvent mixture.
65) According to another particular method of performing the process according to embodiments 58) to 63), the appropriate quantity of compound of formula I-1 is added over a period of time to a solution of the appropriate quantity of DIBAH in a polar aprotic solvent or a polar aprotic solvent mixture.
66) According to another particular sub-embodiment of embodiment 57), a process according to embodiment 57) will comprise the reaction of the compound of formula I-1 with triisopropoxyaluminium in the presence of iPrOH.
67) Preferably, a process according to embodiment 66) will be performed using about 2 equivalents of triisopropoxyaluminium per equivalent of compound of formula I-1.
68) Preferably, a process according to embodiment 66) or 67) will be performed at a temperature of at least 25°C (and more preferably at a temperature of at least 50°C, for example at a temperature of about 80°C).
69) According to yet another particular sub-embodiment of embodiment 57), a process according to embodiment 57) will comprise the reaction of a compound of formula I-1 with dimethylaluminium chloride or diethylaluminium chloride in the presence of iPrOH (and in particular with dimethylaluminium chloride in the presence of iPrOH).
70) Preferably, a process according to embodiment 69) will be performed using about 2 to 3 equivalents of dimethylaluminium chloride or diethylaluminium chloride per equivalent of compound of formula I-1 (and in particular about 3 equivalents of dimethylaluminium chloride per equivalent of compound of formula I-1).
71) Preferably, a process according to embodiment 69) or 70) will be performed at a temperature of at least 20°C (and more preferably at a temperature of at least 30°C, for example at a temperature of about 40°C).
72) Preferably, a process according to one of embodiments 66) to 71) will be performed in the presence of at least 3 equivalents of iPrOH (and notably at least 5 equivalents of iPrOH) per equivalent of compound of formula I-1 (for example in the presence of about 10 equivalents of iPrOH per equivalent of compound of formula I-1).
73) According to yet another sub-variant of the process according to embodiment 40), the compound of formula I-1 will be such that R² represents benzyloxy and a compound of formula I-4 wherein R^{1'} represents a group -CO-R^{2'} wherein R^{2'} represents benzyloxy will be obtained.
74) According to a particular sub-embodiment of embodiment 73), a process according to embodiment 73) will comprise the reaction of the compound of formula I-1 with DIBAH.
75) In a process according to embodiment 74), the reaction will preferably be performed using from 1.5 to 10 equivalents of DIBAH per equivalent of compound of formula I-1, and notably from 2 to 6 equivalents of DIBAH per equivalent of compound of formula I-1.
76) In a process according to embodiment 74), the reaction will more preferably be performed using from 2 to 4 equivalents of DIBAH per equivalent of compound of formula I-1 (and notably about 2.2 equivalents of DIBAH per equivalent of compound of formula I-1).
77) Preferably, the reaction of the compound of formula I-1 with DIBAH in the process according to one of embodiments 74) to 76) will be performed at a temperature of -30°C or below, and notably at a temperature of -65°C or below (for example at a temperature of about -78°C).
78) In a process according to one of embodiments 74) to 77), the polar aprotic solvent or a polar aprotic mixture of solvents will comprise THF (whereby such polar aprotic solvent or the polar aprotic mixture of solvents will notably be selected from THF and a mixture of THF and toluene and more particularly will consist exclusively of THF).
79) According to one particular method of performing the process according to embodiments 74) to 78), the appropriate quantity of DIBAH is added over a period of time to a solution of the appropriate quantity of compound of formula I-1 in a polar aprotic solvent or a polar aprotic solvent mixture.
80) According to another particular method of performing the process according to embodiments 74) to 78), the appropriate quantity of compound of formula I-1 is added over a period of time to a solution of the appropriate quantity of DIBAH in a polar aprotic solvent or a polar aprotic solvent mixture.
81) According to another particular sub-embodiment of embodiment 73), a process according to embodiment 73) will comprise the reaction of the compound of formula I-1 with triisopropoxyaluminium in the presence of iPrOH.
82) Preferably, a process according to embodiment 81) will be performed using about 2 equivalents of triisopropoxyaluminium per equivalent of compound of formula I-1.
83) Preferably, a process according to embodiment 81) or 82) will be performed at a temperature of at least 25°C (and more preferably at a temperature of at least 50°C, for example at a temperature of about 80°C).
84) According to yet another particular sub-embodiment of embodiment 73), a process according to embodiment 73) will comprise the reaction of a compound of formula I-1 with dimethylaluminium chloride or diethylaluminium chloride in the presence of iPrOH (and in particular with dimethylaluminium chloride in the presence of iPrOH).
85) Preferably, a process according to embodiment 84) will be performed using about 2 to 3 equivalents of dimethylaluminium chloride or diethylaluminium chloride per equivalent of compound of formula I-1 (and in particular about 3 equivalents of dimethylaluminium chloride per equivalent of compound of formula I-1).
86) Preferably, a process according to embodiment 84) or 85) will be performed at a temperature of at least 20°C (and more preferably at a temperature of at least 30°C, for example at a temperature of about 40°C).
87) Preferably, a process according to one of embodiments 81) to 86) will be performed in the presence of at least 3 equivalents of iPrOH (and notably at least 5 equivalents of iPrOH) per equivalent of compound of formula I-1 (for example in the presence of about 10 equivalents of iPrOH per equivalent of compound of formula I-1).
88) According to yet another sub-variant of the process according to embodiment 40), the compound of formula I-1 will be such that R² represents 4-methoxybenzyloxy and a compound of formula I-4 wherein R¹ represents a group -CO-R^{2'} wherein R^{2'} represents 4-methoxybenzyloxy will be obtained.
89) According to a particular sub-embodiment of embodiment 88), a process according to embodiment 88) will comprise the reaction of the compound of formula I-1 with DIBAH.
90) In a process according to embodiment 89), the reaction will preferably be performed using from 1.5 to 10 equivalents of DIBAH per equivalent of compound of formula I-1, notably from 2 to 6 equivalents of DIBAH per equivalent of compound of formula I-1, and in particular from 2 to 4 equivalents of DIBAH per equivalent of compound of formula I-1 (for example about 2.2 equivalents of DIBAH per equivalent of compound of formula I-1).
91) Preferably, the reaction of the compound of formula I-1 with DIBAH in the process according to embodiment 89) or 90) will be performed at a temperature of -30°C or below, and notably at a temperature of -65°C or below (for example at a temperature of about - 78°C).
92) In a process according to one of embodiments 89) to 91), the polar aprotic solvent or a polar aprotic mixture of solvents will comprise THF (whereby such polar aprotic solvent or the polar aprotic mixture of solvents will notably be selected from THF and a mixture of THF and toluene and more particularly will consist exclusively of THF).
93) According to one particular method of performing the process according to embodiments 89) to 92), the appropriate quantity of DIBAH is added over a period of time to a solution of the appropriate quantity of compound of formula I-1 in a polar aprotic solvent or a polar aprotic solvent mixture.
94) According to another particular method of performing the process according to embodiments 89) to 92), the appropriate quantity of compound of formula I-1 is added over a period of time to a solution of the appropriate quantity of DIBAH in a polar aprotic solvent or a polar aprotic solvent mixture.
95) According to another particular sub-embodiment of embodiment 88), a process according to embodiment 88) will comprise the reaction of the compound of formula I-1 with triisopropoxyaluminium in the presence of iPrOH.
96) Preferably, a process according to embodiment 95) will be performed using about 2 equivalents of triisopropoxyaluminium per equivalent of compound of formula I-1.
97) Preferably, a process according to embodiment 95) or 96) will be performed at a temperature of at least 25°C (and more preferably at a temperature of at least 50°C, for example at a temperature of about 80°C).
98) According to yet another particular sub-embodiment of embodiment 88), a process according to embodiment 88) will comprise the reaction of a compound of formula I-1 with dimethylaluminium chloride or dietlrylaluminium chloride in the presence of iPrOH (and in particular with dimethylaluminium chloride in the presence of iPrOH).
99) Preferably, a process according to embodiment 98) will be performed using about 2 to 3 equivalents of dimethylaluminium chloride or diethylaluminium chloride per equivalent of compound of formula I-1 (and in particular about 3 equivalents of dimethylaluminium chloride per equivalent of compound of formula I-1).
100) Preferably, a process according to embodiment 98) or 99) will be performed at a temperature of at least 20°C (and more preferably at a temperature of at least 30°C, for example at a temperature of about 40°C).
101) Preferably, a process according to one of embodiments 95) to 100) will be performed in the presence of at least 3 equivalents of iPrOH (and notably at least 5 equivalents of iPrOH) per equivalent of compound of formula I-1 (for example in the presence of about 10 equivalents of iPrOH per equivalent of compound of formula I-1).

The compounds of formula I-1, I-2 or I-3_{BOC} required for performing the manufacturing processes described in the preceding embodiments can be prepared as described hereafter.

### PREPARATION OF STARTING MATERIALS

### Compounds of formula I-1:

The compound of formula I-1 wherein R¹ is *tert*-butoxy can be prepared by a process comprising the reaction of the compound of formula I-2 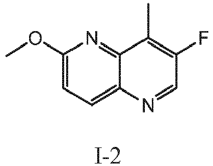 with the compound of formula I-3_{BOC} 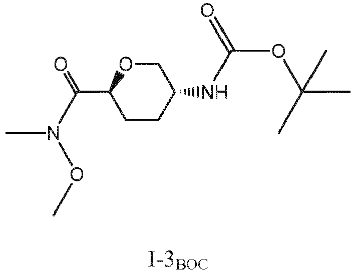 said reaction being performed in the presence of LDA, LiHMDS or a mixture thereof in a polar aprotic solvent or a polar aprotic mixture of solvents at a temperature of 20°C or below (and preferably at a temperature of 10°C or below).

The compounds of formula I-1 wherein R¹ is trifluoromethylcarbonyl, benzyloxycarbonyl or 4-methoxybenzyloxycarbonyl can be obtained by the following two step process:
1. removing the Boc protecting group using a strong acid (such as HC1) in an alkanol solvent (such as isopropanol); and
2. reacting the intermediate free amine obtained after the first step either with trifluoroacetic anhydride (if R¹ is trifluoromethyl), or with benzyl chloroformate (if R¹ is benzyloxy) or 4-methoxybenzyl chloroformate (if R¹ is 4-methoxybenzyloxy) in the presence of a base such as NaOH, TEA, DMAP or imidazole.

### Compound of formula I-2:

The compound of formula I-2 can be prepared for example as described in WO 2010/067332.

### Compounds of formula I-3_{BOC}:

The compound of formula I-3_{BOC} can be prepared starting from the corresponding known carboxylic acid derivative of formula I-6 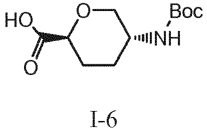 the preparation of which is described in Kriek et al., Eur. J. Org. Chem. (2003), 13, 2418-2427.

### ABBREVIATIONS AND TERMS USED IN THIS TEXT

### Abbreviations:

The following abbreviations are used throughout the specification and the examples:
- aq.: aqueous
- Boc: *tert*-butoxycarbonyl
- DIBAH: diisobutyl aluminium hydride
- DCM: dichloromethane
- DEA: diethylamine
- DMAP: 4-dimethylaminopyridine
- DME: 1,2-dimethoxyethane
- DMF: dimethylformamide
- DMSO: dimethylsulfoxide
- d6-DMSO: perdeuterated dimethylsulfoxide
- eq.: equivalent(s)
- EA: ethyl acetate
- EtOH: ethanol
- Hept: heptane
- iPrOAc: isopropyl acetate
- iPrOH: isopropanol
- KHMDS: potassium hexamethyldisilazide
- LC-MS: liquid chromatography - mass spectroscopy
- LiHMDS: lithium hexamethyldisilazide
- min: minute(s)
- org.: organic
- RT: room temperature

- TBME: *tert*-butyl methyl ether
- TEA: triethylamine
- TEMPO: 2,2,6,6-tetramethyl-1-piperidinyloxy
- TFA: trifluoroacetic acid
- THF: tetrahydrofurane
- TLC: thin layer chromatography
- t_{R}: retention time

### Definitions of particular terms used in this text:

The following paragraphs provide definitions of the various chemical moieties for the compounds according to the invention as well as other particular terms used in this text and are intended to apply uniformly throughout the specification and claims, unless an otherwise expressly set out definition provides a broader or narrower definition:
❖ The term "alkyl", used alone or in combination, refers to a saturated straight or branched chain alkyl group containing from one to four carbon atoms. Representative examples of alkyl groups include methyl, ethyl, propyl, *iso*-propyl, *n*-butyl, *iso*-butyl, *sec*-butyl and *tert*-butyl. The term "(C₁-Cₓ)alkyl" (x being an integer) refers to a straight or branched chain alkyl group containing 1 to x carbon atoms.
❖ The term "alkoxy", used alone or in combination, refers to a saturated straight or branched chain alkoxy group containing from one to four carbon atoms. Representative examples of alkoxy groups include methoxy, ethoxy, propoxy, *iso*-propoxy, *n*-butoxy, *iso*-butoxy, *sec*-butoxy and *tert*-butoxy. The term "(C₁-Cₓ)alkoxy" refers to a straight or branched chain alkoxy group containing 1 to x carbon atoms.
❖ The term "alkanol" refers to an aliphatic primary, secondary or tertiary alcohol containing from one to six carbon atoms and one hydroxy group. The term "(C₁-Cₓ)alkanol" refers to an aliphatic primary, secondary or tertiary alcohol containing 1 to x carbon atoms and one hydroxyl group. Examples of alkanols include methanol, ethanol and isopropanol.
❖ The term "stereoselective", when used regarding the preparation of a particular stereomer, means that the preparation of this stereomer leads to a mixture wherein the desired particular stereomer is obtained in a relative proportion of at least 4:1, preferably of at least 7:1 and more preferably of at least 95:5 with respect to the non-desired stereomer(s).
❖ The term "polar aprotic solvent" refers to a solvent which does not display hydrogen bonding, does not have an acidic hydrogen but is able to stabilise ions. Representative examples of polar aprotic solvents include DCM, EA, iPrOAc, THF, 2-methyl-tetrahydrofurane, DME, DMF, dioxane, diethyl ether, *tert*-butyl methyl ether or cyclopentyl methyl ether.
❖ The term "polar aprotic mixture of solvents" refers to a mixture of solvents which includes at least one polar aprotic solvent as previously defined and at least another aprotic solvent (which may be polar or apolar). Representative examples of polar aprotic mixtures of solvents include, but are not limited to: a mixture of two solvents selected from the group consisting of DCM, EA, iPrOAc, THF, DME, DMF, dioxane and diethyl ether; a mixture of toluene with one or more of DCM, EA, iPrOAc, THF, 2-methyl-tetrahydrofurane, DME, DMF, dioxane or diethyl ether; a mixture of hexane with one or more of DCM, EA, iPrOAc, THF, DME, DMF, dioxane, diethyl ether, *tert*-butyl methyl ether or cyclopentyl methyl ether; a mixture of Hept with one or more of DCM, EA, iPrOAc, THF, 2-methyl-tetrahydrofurane, DME, DMF, dioxane, diethyl ether, *tert*-butyl methyl ether or cyclopentyl methyl ether; and a mixture of toluene with hexane or Hept and one or more of DCM, EA, iPrOAc, THF, 2-methyl-tetrahydrofurane, DME, DMF, dioxane, diethyl ether, *tert*-butyl methyl ether or cyclopentyl methyl ether.
❖ The term "over a period of time" used regarding a particular process or operation refers to a process or operation that is performed over a period not shorter than 1 min (and preferably not shorter than 5 min).
❖ The term "room temperature" as used herein refers to a temperature of from 20 to 30°C, and preferably 25°C.
❖ Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C.

Particular embodiments of the invention are described in the following Examples, which serve to illustrate the invention in more detail.

### EXAMPLES

All temperatures given are internal temperatures and are stated in °C. Compounds were characterized by ¹H-NMR (400 MHz) or ¹³C-NMR (100 MHz) (Bruker; chemical shifts δ are given in ppm relative to the solvent used; multiplicities: s = singlet, d = doublet, t = triplet; p = pentuplet, hex = hexet, hept = heptet, m = multiplet, br. = broad, coupling constants are given in Hz); by GC-MS, by LC-MS methods 1 and 2 (Finnigan Navigator with HP 1100 Binary Pump and DAD); by chiral HPLC (Dionex HPG-3400SD Bin pump, Dionex DAD-3000). Unless stated otherwise, yields are given as is.

### Parameters of GC-MS, LC-MS and chiral HPLC methods:

### GC-MS:

| | |
|---|---|
| Injection volume: | 1 µL |
| Column: | Zebron ZB-5-MS, 15 m x 0.25 mm ID, 0.25 µm film |
| Column flow: | 2 ml/min |
| Carrier gas: | Helium |
| Split ratio: | 20 |
| SSL inlet temperature: | 200°C |
| Temperature gradient: | 60-300°C from 0 to 4.0 min |
| | 300°C isotherm from 4.0 to 5.0 min |
| Ionization: | Chemical ionization with CH₄ as reagent gas |

*LC-MS method 1:*

| | | |
|---|---|---|
| Injection volume: | | 2 µL |
| Column: | Kinetex 2.6 µm C18, 2.1 x 50 mm | |
| Column flow: | 1 ml/min | |
| Eluent: | Eluent A: water, 0.08% TFA | |
| | Eluent B: acetonitrile, 0.012% TFA | |
| Gradient: | 2.0 min | 95% B |
| | 2.8 min | 95% B |
| | 3.0 min | 5% B |
| Temperature: | 40°C | |
| Detection: | 210 nm | |

*LC-MS method 2:*

| | | |
|---|---|---|
| Injection volume: | 2 µL | |
| Column: | Zorbax SB-Aq, 3.5 µm, 4.6 x 50 mm | |
| Column flow: | 4.5 ml/min | |
| Eluent: | Eluent A: water, 0.04% TFA | |
| | Eluent B: acetonitrile | |
| Gradient: | 1.0 min | 95% B |
| | 1.5 min | 95% B |
| | 2.0 min | 5% B |
| Temperature: | 40°C | |
| Detection: | 210 nm | |

*Chiral HPLC method 1:*

| | |
|---|---|
| Injection volume: | 5 µL |
| Column: | ChiralPak AD-H, 4.6 x 250 mm, 5 µm |
| Column flow: | 0.8 ml/min |
| Eluent: | Hept (40%) / EtOH (60%) |
| Concentration: | 1.3 mg / mL Hept / EtOH 1 : 1 |
| Pressure: | 92 bars |
| Temperature: | 25°C |
| Detection: | 210 nm |

*Chiral HPLC method 2:*

| | |
|---|---|
| Injection volume: | 2 µL |
| Column: | ChiralPak AD-H, 150 mm x 4.6 mm, 5 µm |
| Column flow: | 1 ml/min |
| Eluent: | EtOH / MeOH /DEA (80 / 20 / 01) |
| Concentration: | 1.3 mg / mL Hept / EtOH 1 : 1 |
| Pressure: | 75 bar |
| Temperature: | 40°C |
| Detection: | 323 nm |

### General procedures:

### General Procedure A (reduction using D/BAH):

To a solution of the ketone derivative (100 mg) in THF (1 mL) at -78°C is added with caution a 1.0*M* solution of DIBAH in THF (4.5 eq.) at such a rate that the internal temperature does not exceed -65°C. The resulting solution is stirred at -78°C for 1 h.

### General Procedure B (reduction using aluminium triisopropoxide):

To a solution of the ketone derivative (100 mg) in iPrOAc (2 mL) is added iPrOH (0.180 mL, 2.38 mmol, 10 eq.) followed by aluminium triisopropoxide (97 mg; 0.48 mmol, 2 eq). The resulting solution is stirred at 80°C for 1 h.

### General Procedure C (reduction using dimethylaluminium chloride):

To a solution of the ketone derivative (100 mg) in toluene (2 mL) is added iPrOH (0.180 mL, 2.38 mmol, 10 eq.) followed by a 1.0*M* solution of dimethylaluminum chloride in hexanes (0.72 mL; 0.72 mmol, 3 eq.).

### Reference Example 1: non-stereoselective preparation of tert-butyl ((3R,6S)-6-((S)-2-(3-fluoro-6-methoxy-1,5-naphthyridin-4-yl)-1-hydroxyethyl)tetrahydro-2H-pyran-3-yl)carbamate:

NaBH₄ (36 mg, 0.95 mmol, 2 eq.) was added at -78°C to a solution of *tert-butyl* ((*3R,6S*)-6-(2-(3-fluoro-6-methoxy-1,5-naphtliyridin-4-yl)acetyl)tetrahydro-2*H*-pyran-3-yl)carbamate (200 mg) in MeOH (5 mL). The resulting mixture was stirred at -78°C for 15 h. It was quenched by the addition of water. The mixture was extracted twice with EA. The combined org. extracts were concentrated under vacuum at a temperature of 50°C, affording the title compound as a crude yellow solid (180 mg; 90% yield) consisting of a 3.8:1 mixture of diastereoisomers (determined by chiral HPLC method 1).

The major product has NMR data equivalent to those obtained for the product of Example 4, Variant 1 described hereafter.

### Reference Example 2: non-stereoselective preparation of tert-butyl ((3R,6S)-6-((S)-2-(3-fluoro-6-methoxy-1,5-naphthyridin-4-yl)-1-hydroxyethyl)tetrahydro-2H-pyran-3-yl)carbamate:

A 1.0*M* solution of LiAlH₄ in THF (14.3 mL, 14.3 mmol, 2 eq.) was added dropwise to a solution of *tert-butyl* ((*3R,6S*)-6-(2-(3-fluoro-6-methoxy-1,5-naphthyridin-4-yl)acetyl)tetrahydro-2*H*-pyran-3-yl)carbamate (3 g, 7.15 mmol) in THF (20 mL) at -78°C. After 1 h, the reaction mixture was warmed up to 0°C and quenched by the addition of a solution of water in THF. Water was added followed by citric acid and the mixture was extracted twice with ethyl acetate. The combined org. extracts were concentrated under vacuum at a temperature of 50°C, affording the title compound as a crude yellow solid (2.82 g; 93% yield) consisting of a 1.2:1 mixture of diastereoisomers (determined by chiral HPLC method 1).

The major product has NMR data equivalent to those obtained for the product of Example 4, Variant 1 described hereafter.

### Reference Example 3: non-stereoselective preparation of tert-butyl ((3R,6S)-6-((S)-2-(3-fluoro-6-methoxy-1,5-naphthyridin-4-yl)-1-hydroxyethyl)tetrahydro-2H-pyran-3-yl)carbamate:

Sodium bis(2-methoxyethoxy)aluminium hydride was added dropwise to a solution of *tert*-butyl ((*3R,6S*)-6-(2-(3-fluoro-6-metboxy-1,5-naphthyridin-4-yl)acetyl)tetrahydro-2*H*-pyran-3-yl)carbamate (500 mg) in toluene (5 mL) at -20°C. The mixture was stirred at -20°C. After 2 h, the reaction mixture was warmed up to 0°C and quenched by the addition of a solution of water in THF. Water was added followed by citric acid and the mixture was extracted twice with toluene. The combined org. extracts were concentrated under vacuum at a temperature of 50°C, affording the title compound as a crude yellow solid (500 g; 100% yield) consisting of a 1:1.2 mixture of diastereoisomers (determined by chiral HPLC method 1).

The minor product has NMR data equivalent to those obtained for the product of Example 4, Variant 1 described hereafter.

### Example 1: tert-butyl ((3R,6S)-6-(2-(3-fluoro-6-methoxy-1,5-naphthyridin-4-yl)acetyl)tetrahydro-2H pyran-3-yl)carbamate:

### 1.i. (2S,5R)-5-((tert-butoxycarbonyl)amino)tetrahydro-2H-pyran-2-carboxylic acid:

A reactor was charged with *tert*-butyl ((*3R,6S*)-6-(hydroxymethyl)tetrahydro-2*H*-pyran-3-yl)carbamate (1700 g; 1 eq.; prepared as described in Kriek et al., Eur. J. Org. Chem. (2003), 13, 2418-2427), NaHCO₃ (1241 g, 2 eq.), KBr (87 g, 0.1 eq.), TEMPO (17.6 g, 0.015 eq.), Aliquat 336 (17 mL, 0.005 eq.) and iPrOAc (17 L). The suspension was cooled to 0 °C and 10 % NaOCl solution (10.2 L, 2.33 eq.) was added keeping the internal temperature below 5°C. The suspension was stirred at 10°C for 90 min. No starting material could be detected by TLC (visual control by ninhydrin stain). A 40% NaHSO₃ solution (1.5 L) was added at 12°C. 25% HCl (2.5 L) was then added at 15°C. The mixture was heated to 20°C. The aq. layer was separated and extracted with iPrOAc (8.5 L). The combined org. layers were washed with water (15 L). The org. layer was concentrated at a jacket temperature of 100 °C and reduced pressure. 18 L solvent were removed, the residue was cooled to 65°C and Hept (14 L) was added. The resulting suspension was cooled to -6 °C and filtered. The product was dried on a 20 L rotary evaporator to yield the title compound as a white solid (1406 g; 78% yield).
¹H-NMR (d6-DMSO): δ = 12.64 (m, 1H), 6.85 (m, 1H), 3.84 (m, 1H), 3.76 (m, 1H), 3.32 (m, 1H), 3.01 (t, J = 10.5 Hz, 1H), 1.92 (m, 2H), 1.53 (m, 2H), 1.39 (s, 9H).
GC-MS: t_{R} = 3.49 min; [M+1]⁺ = 246.

### 1. ii. tert-butyl ((3R, 6S)-6-(methoxy(methyl)carbamoyl)tetrahydro-2H-pyran-3-yl)carbamate:

A reactor was charged with intermediate 1.i (1098 g, 1 eq.) and DCM (8.8 L). Pivaloylchloride (540 g, 1 eq.) was added at 20 °C. 4-methylmorpholine (475 g, 1.05 eq.) was added at 20 °C with cooling. *N*,*O*-dimethylhydroxylamine (440 g, 1.0 eq.) was added. 4-methylmorpholine (475 g, 1.05 eq.) was added at 20 °C with cooling. The suspension was stirred for 30 min at 20°C. Water (5 L) and 25% HCl (100 mL) were added. The org. layer was separated and washed with 1*N* NaOH (5 L) and water (5 L). A solvent switch from DCM to iPrOAc was performed at 65°C and reduced pressure. 8 L solvent were distilled off and iPrOAc (2 L) was added during distillation. Hept (8 L) was added at 59°C. The mixture was heated to 100°C external temperature and water was removed by azeotropic drying under reduced pressure. After the water was removed a white suspension resulted, which was cooled to 6 °C and filtered. The collected solid was rinsed with Hept (2 L), transferred into a beaker and dried by standing in a hood overnight to yield an off-white solid (990 g; 75% yield).
¹H-NMR (d6-DMSO): δ = 6.82 (d, J = 7.6 Hz, 1H), 4.06 (m, 1H), 3.84 (m, 1H), 3.68 (s, 3H), 3.33 (m, 1H), 3.11 (s, 3H), 3.03 (m, 1H), 1.90 (m, 1H), 1.71 (m, 1H), 1.56 (m, 2H), 1.39 (s, 9H).
GC-MS: t_{R} = 3.82 min; [M+1]⁺ = 289, 233.

### 1. iii. tert-butyl ((3R,6S)-6-(2-(3-fluoro-6-methoxy-1,5-naphthyridin-4-yl)acetyl)tetrachydro-2H-pyran-3-yl)carbamate:

### Variant 1 (LiHMDS):

A reactor was charged with 7-fluoro-2-methoxy-8-methyl-1,5-naphthyridine (500 g, 1 eq.), intermediate 1.ii (888 g, 1.2 eq.) and THF (10 L). The solution was cooled to -10°C and 1*M* LiHMDS in THF (8 L, 3 eq.) was added over a period of 30 min, keeping the internal temperature below -5°C. The reaction mixture was stirred for 3 h at 0°C and in-process-control by LC-MS showed less than 3% of 7-fluoro-2-methoxy-8-methyl-1,5-naphthyridine. A solution of citric acid monohydrate (1.8 kg, 3.3 eq.) in water (5 L) was added to the reaction mixture at 0 to 20°C. The aq. layer was discarded. The org. layer was washed with a mixture of brine (5 L) and a 20% citric acid solution (1 L). The org. layer was concentrated at 60°C under reduced pressure (16 L solvent removed). To the residue was added TBME (10 L) at 25 °C. The org. layer was washed with 1*N* HCl (10 L) and water (5 L). The org. layer was concentrated at 60 °C and reduced pressure (7.5 L solvent removed). Hept (10 L) was added at 60°C and 1 L of solvent was distilled off under reduced pressure. At that point the product started to crystallize. The suspension was aged at 68°C for 15 min and then cooled down to 10°C. The suspension was filtered; the product on the Nutsche filter was washed with Hept (5 L). The product was dried in a drying oven at 50°C and reduced pressure overnight to yield the title compound as a yellow solid (823 g; 75% yield).
¹H-NMR (CDCl₃): δ = 8.69 (s, 1H), 8.21 (d, J = 9.1 Hz, 1H), 7.08 (d, J = 9.1 Hz, 1H), 4.50 (m, 2H), 4.37 (m, 1H), 4.28 (m, 1H), 4.03 (s, 3H), 3.94 (m, 1H), 3.75 (m, 1H), 3.15 (t, J = 10.5 Hz, 1H), 2.20 (m, 1H), 2.12 (m, 1H), 1.74 (m, 1H), 1.48 (s, 9H), 1.41 (m, 1H). LC-MS (method 1): t_{R} = 1.65 min; [M+1]⁺ = 420.

### Variant 2 (LDA):

A 10 mL flask was charged with 7-fluoro-2-methoxy-8-methyl-1,5-naphthyridine (167 mg, 1 eq.), intermediate 1.ii (250 mg, 1.0 eq.) and THF (5 mL). The solution was cooled to - 78°C and a solution of LDA (3 eq.) in THF (prepared from 2.5*M* HexLi in hexane (1 mL) and diisopropylamine (0.371 ml) in THF (3 mL)) was added at -20°C. The mixture was allowed to warm to 0°C over a period of 2 h. To the mixture was added a 20% aq. solution of citric acid (3 mL) and TBME (10 mL). The aq. layer was discarded and the org. layer was evaporated to dryness. The residue was purified by flash chromatography using silica gel (30 g) and EA / Hept 1:2 (v:v). A yellow oil (70 mg, 19% yield) was obtained. The product has NMR data equivalent to those obtained for the product of Variant 1.

### Example 2: benzyl ((3R,6S)-6-(2-(3-fluoro-6-methoxy-1,5-naphthyridin-4-yl)acetyl)tetrahydro-2H-pyran-3-yl)carbamate:

### 2.i. 1-((2S,5R)-5-aminotetrahydro-2H-puran-2-yl)-2-(3-fluoro-6-methoxy-1, 5-naphthyriclin-4-yl)ethcinone:

The compound of Example 1 (5 g, 11.9 mmol) was suspended in iPrOH (50 mL). The mixture was stirred at 40°C. 5*M* HCl in iPrOH (25 mL, 119.0 mmol, 10 eq.) was added dropwise. The resulting suspension was stirred at 40°C for 3 h. It was cooled down to 4°C, filtered, rinsed with iPrOH and dried under vacuum to yield a light yellow solid (4.24 g, 100% yield).
¹H-NMR (d6-DMSO): δ = 8.84 (s, 1H), 8.38 (br s, 2H), 8.32 (d, J = 9.1 Hz, 1H), 7.25 (d, J = 9.1 Hz, 1H), 4.52-4.40 (m, 2H), 4.23-4.15 (m, 2H), 3.99 (s, 3H), 3.50 (t, J = 10.6 Hz, 1H), 3.24-3.15 (m, 1H), 2.19-2.16 (m, 1H), 2.10-2.05 (m, 1H), 1.78-1.53 (m, 2H).
LC-MS (method 2): t_{R} = 0.58 min; [M+1]⁺ = 320.

### 2.ii. Benzyl ((3R,6S)-6-(2-(3-fluoro-6-methoxy-1,5-naphthyridin-4-yl)acetyl)tetrahydro-2H-pyran-3-yl)carbamate:

Solid NaHCO₃ (708 mg, 8.43 mmol, 3 eq) was added cautiously to a cold (4°C) solution of intermediate 2.i (1 g, 2.81 mmol) in acetone (5 mL), THF (5 mL) and water (10 mL). Benzylchloroformate (0.48 mL, 3.37 mmol, 1.2 eq.) was then added dropwise. It was stirred at RT for 1 h 30. The volatiles were removed under vacuum. The residue was partitioned between DCM and water. The aq. phase was extracted with DCM and the combined org. extracts were dried over Na₂SO₄ and concentrated to dryness to afford a yellow solid (1.1 g). This material was dissolved in hot EA (7 mL), allowed to cool down to RT, filtered and dried to yield an off-white solid (370 mg, 29% yield).
¹H-NMR (d6-DMSO): δ = 8.83 (s, 1H), 8.31 (d, J = 9.1 Hz, 1H), 7.24 (d, J = 9.1 Hz, 1H), 7.40-7.32 (m, 6H), 5.08-5.00 (m, 2H), 4.52-4.38 (m, 2H), 4.07-4.01 (m, 2H), 3.98 (s, 3H), 3.54-3.48 (m, 1H), 3.20-3.15 (m 1H), 2.02-1.99 (m, 2H), 1.65-1.49 (m, 2H).
LC-MS (method 2): t_{R} = 0.94 min; [M+1]⁺ = 454.

### Example 3: 2,2,2-trifluoro-N-((3R,6S)-6-(2-(3-fluoro-6-methoxy-1,5-naphthyridin-4-yl)acetyl)tetrahydro-2H-pyran-3-yl)acetamide:

Trifluoroacetic acid anhydride (0.47 mL, 3.37 mmol, 1.2 eq.) was added dropwise to a solution of intermediate 2.i (1 g, 2.81 mmol) and Et₃N (1.17 mL, 8.43 mmol, 3 eq.) in DCM (10 mL) at RT. The resulting mixture was stirred at RT for 1 h 30. Water was added and the layers were separated. The aq. phase was extracted with DCM. The combined org. phases were washed with a saturated aq. solution of NaHCO₃, dried over Na₂SO₄ and evaporated to dryness. The crude residue was dissolved in hot TBME (7 mL) and hot EA (1 mL). The mixture was then allowed to cool down to 4°C. It was diluted with TBME, filtered and dried to yield a beige solid (500 mg, 43% yield).
¹H-NMR (d6-DMSO): δ = 9.38-9.36 (m, 1H), 8.83 (s, 1H), 8.31 (d, J = 9.0 Hz, 1 H), 7.24 (d, J = 9.1 Hz, 1H), 4.53-4.39 (m, 2H), 4.14-4.11 (m, 1H), 4.06-4.02 (m, 1H), 3.99 (s, 3H), 3.91-3.79 (m, 1H), 3.37-3.34 (m, 1H), 2.07-2.01 (m, 2H), 1.79-1.60 (m, 2H).
LC-MS (method 2): t_{R} = 0.88 min, [M+1]⁺ = 416.

### Example 4: tert-butyl ((3R,6S)-6-((S)-2-(3-fluoro-6-methoxy-1,5-naphthyridin-4-yl)-1-hydroxyethyl)tetrahydro-2H-pyran-3-yl)carbamate:

### Variant 1:

A 2.5 L sulfier flask was charged with the compound of Example 1 (190 g, 1 eq.) and THF (950 mL). 1*M* DIBAH in THF (1 L, 2.2 eq.) was added over a period of 30 min at an internal temperature below -68°C. The reaction mixture was stirred for 1 h at -75 °C and in-process-control by LC-MS showed less than 2% of compound of Example 1. A solution of citric acid monohydrate (230 g, 2.4 eq.) in water (700 mL) was heated to 45°C in a 4 L flask. The cold reaction mixture was added to the citric acid solution over a period of 30 min. The mixture was stirred at 55°C for 30 min. The aq. layer was separated. The org. layer was combined with a second batch, carried out in the same manner with equal amount of starting materials. The combined org. layers (4.4 L) were concentrated under reduced pressure on a rotary evaporator to a volume of about 1.4 L. iPrOAc (2.8 L) was added and the solution was washed with water (2 x 2 L). The ratio of the title compound to the epimeric homobenzylic alcohol was 95:5 according to chiral HPLC method 1. The org. layer was concentrated at 110°C and 2.2 L solvent were removed. The residue was cooled to 84°C to yield a thick suspension, which was diluted with Hept (2 L). The mixture was aged for 30 min at 80 °C and then cooled to 10°C. The suspension was filtered over a nutsche and the product was washed with Hept (600 mL). The alcohol was dried on a rotary evaporator at 60°C under a pressure of 40 mbar to yield an off-white solid (294 g; 77% yield).
¹H-NMR (d6-DMSO): δ = 8.76 (s, 1H), 8.28 (d, J = 9.0 Hz, 1H), 7.24 (d, J = 9.0 Hz, 1H), 6.76 (m, 1H), 4.65 (d, J = 6.1 Hz, 1H), 4.04 (s, 3H), 3.93 (m, 1H), 3.84 (m, 1H), 3.31 (m, 2H), 3.13 (m, 3H), 2.95 (t, J = 10.6 Hz, 1H), 1.90 (m, 1H), 1.70 (m, 1H), 1.57 (m, 1H), 1.38 (m, 9H).
Diastereomeric ratio (S:R): 98:2 (determined by chiral HPLC method 1).
LC-MS (method 1): t_{R} = 1.54 min; [M+1]⁺ = 422.

### Variant 2:

General Procedure A was performed on the compound of Example 1, except that 4.0 eq. of DIBAH were used. After stirring, the reaction mixture was warmed to 0°C and diluted with TBME. Water was added followed by citric acid and the mixture was extracted twice with DCM. The combined org. extracts were concentrated under vacuum at a temperature of 50°C, affording the title compound as a crude white solid (100 mg; 100% yield) consisting of a 26:1 mixture of diastereoisomers (determined by chiral HPLC method 1). The experiment was repeated using the same protocol. The title compound was again obtained as a crude white solid (100 mg; 100% yield), but this time consisting of a 32:1 mixture of diastereoisomers (determined by chiral HPLC method 1).

The products obtained have in both cases NMR data equivalent to those obtained for the product of Variant 1.

### Variant 3:

General Procedure A was performed on the compound of Example 1, using however a mixture of THF (0.4 mL) and toluene (0.7 mL) to dissolve the compound of Example 1 and 4.0 eq. of DIBAH (instead of 4.5). After stirring, the reaction mixture was warmed up to 0°C and diluted with TBME. Water was added followed by citric acid and the mixture was extracted twice with DCM. The combined org. extracts were concentrated under vacuum at a temperature of 50°C, affording the title compound as a crude white solid (87 mg; 87% yield) consisting of a 15:1 mixture of diastereoisomers (determined by chiral HPLC method 1).

The product has NMR data equivalent to those obtained for the product of Variant 1.

### Variant 4:

A solution of the compound of Example 1 (12 g, 28.6 mmol) in THF (60 mL) was slowly added to a solution of 1.0*M* solution of DIBAH in THF (115 mL, 115 mmol , 4.0 eq,) at - 78°C at such a rate that the internal temperature did not exceed -65°C. The resulting solution was stirred at -78°C for 1 h. After stirring, the reaction mixture was warmed up to 0°C and diluted with TBME. Water was added followed by citric acid and the mixture was extracted twice with DCM. The combined org. extracts were concentrated under vacuum at a temperature of 50°C, affording the title compound as a crude white solid (9.8 g; 82% yield) consisting of a 28 / 1 mixture of diastereoisomers (determined by chiral HPLC method 1).

The product has NMR data equivalent to those obtained for the product of Variant 1.

### Variant 5:

A 2.5 L sulfier flask was charged with the compound of Example 1 (140 g, 1 eq.) and THF (700 mL). 1*M* DIBAH in THF (1 L, 3 eq.) was added over a period of 90 min at an internal temperature below -68°C. The reaction mixture was stirred for 1 h at -75°C and in-process-control by LC-MS showed less than 2% of compound of Example 1. To a solution of citric acid monohydrate (217 g, 3 eq.) in water (700 mL) was added the cold reaction mixture over a period of 30 min at 20-30°C. The mixture was stirred at 24°C for 30 min. The aq. layer was separated. The org. layer was concentrated under reduced pressure on a rotary evaporator to a volume of about 0.7 L. iPrOAc (1 L) was added and the solution was washed with water (2 x 1 L). The ratio of the title compound to the epimeric homobenzylic alcohol was 95.5:4.5 according chiral HPLC method 1. The org. layer was concentrated at 110°C and 0.7 L solvent were removed. The residue was cooled to 53°C to yield a thick suspension, which was diluted with Hept (0.6 L). The mixture was aged for 30 min at 80°C and then cooled to 20°C. The suspension was filtered over a nutsche and the product was washed with Hept (400 mL). The alcohol was dried on a rotary evaporator at 60°C under a pressure of 40 mbar to yield an off-white solid (90.3 g; 64% yield).

Diastereomeric ratio (S:R): 100:0 (determined by chiral HPLC method 1 as described above).

### Variant 6:

General Procedure B was performed on the compound of Example 1. The mixture was cooled and quenched by the addition of water in iPrOH followed by the addition of 10% aq. citric acid. It was stirred for 1 h. The mixture was diluted with EA. The layers were separated and the aq. layer was back-extracted with EA. The combined org. layers were washed with aq. NaHCO₃ and water and concentrated to dryness to yield the crude title compound (86 mg, 86% yield) as a yellow oil consisting of a 7:1 mixture of diastereisomers (determined by chiral HPLC method 1).

The product has NMR data equivalent to those obtained for the product of Variant 1.

### Variant 7:

The same procedure as that used for Variant 6 was reproduced. This time the crude title compound (100 mg, 100% yield) was obtained as a yellow oil consisting of a 7.2:1 mixture of diastereisomers (determined by chiral HPLC method 1).

The product has NMR data equivalent to those obtained for the product of Variant 1.

### Variant 8:

General Procedure C was performed on the compound of Example 1. The resulting solution was stirred at 40°C (external temperature) for 3 h. The mixture was cooled down and quenched by the addition of water in iPrOH followed by the addition of 10% aq. citric acid. It was stirred for 1 h. The mixture was diluted with EA. The layers were separated and the aq. layer was back-extracted with EA. The combined org. layers were washed with water and concentrated to dryness to yield the title compound (86 mg, 86% yield) as a yellow oil consisting of a 9:1 mixture of diastereisomers (determined by chiral HPLC method 1).

The product has NMR data equivalent to those obtained for the product of Variant 1.

### Variant 9:

General Procedure C was performed on the compound of Example 1. The resulting solution was stirred at RT for 3 h. The mixture was quenched by the addition of water in iPrOH followed by the addition of 10% aq. citric acid. It was stirred for 1 h. The mixture was diluted with EA. The layers were separated and the aq. layer was back-extracted with EA. The combined org. layers were washed with water and concentrated to dryness to yield the crude title compound (92 mg, 92% yield) as a yellow oil consisting of a 7:1 mixture of diastereisomers (determined by chiral HPLC method 1).

The product has NMR data equivalent to those obtained for the product of Variant 1.

### Variant 10:

General Procedure A was performed on the compound of Example 1, using however DCM (2 mL) to dissolve the compound of Example 1 and a 1.0*M* solution of DIBAH in DCM (4.0 eq.) (instead of a 1.0*M* solution of DIBAH in THF (4.5 eq.)). After stirring, the reaction mixture was warmed up to 0°C. Water was added followed by citric acid and the mixture was extracted twice with EA. The combined org. extracts were concentrated under vacuum at a temperature of 50°C, affording the title compound as a crude yellow solid (95 mg; 95% yield) consisting of a 4:1 mixture of diastereoisomers (determined by chiral HPLC method 1).

The product has NMR data equivalent to those obtained for the product of Variant 1.

### Variant 11:

To a solution of the compound of Example 1 (100 mg) in toluene (2 mL) was added iPrOH (0.18 mL, 2.38 mmol, 10 eq.) followed by a 1.0*M* solution of diethylaluminum chloride in hexanes (0.48 mL, 0.48 mmol, 2 eq.). The resulting solution was stirred at 60°C (external temperature) for 15 h. The mixture was cooled down and quenched by the addition of water in iPrOH followed by the addition of 10% aq. citric acid. It was stirred for 1 h. The mixture was diluted with EA. The layers were separated and the aq. layer was back-extracted with EA. The combined org. layers were washed with water and concentrated to dryness to yield the title compound (59 mg, 59% yield) as a yellow oil consisting of a 4.3:1 mixture of diastereisomers (determined by chiral HPLC method 1).

The product has NMR data equivalent to those obtained for the product of Variant 1.

### Example 5: benzyl ((3R,6S)-6-((S)-2-(3-fluoro-6-methoxy-1,5-naphthyridin-4-yl)-1-hydroxyethyl)tetrahydro-2H-pyran-3-yl)carbamate:

General Procedure A was performed on the compound of Example 2. After stirring, the rection mixture was warmed to 0°C and diluted with TBME. Water was added followed by citric acid and the mixture was extracted twice with DCM. The combined org. extracts were concentrated under vacuum at a temperature of 50°C, affording the title compound as a crude white solid (70 mg; 70% yield) consisting of a 85:15 mixture of diastereoisomers (determined by chiral HPLC method 2).
¹H-NMR (d6-DMSO): δ = 8.75 (br. s, 1H), 8.28 (d, J = 9.1 Hz, 1H), 7.24 (d, J = 9.1 Hz, 1H), 7.41-7.30 (m, 5H), 7.21-7.17 (m, 1H), 5.05-4.98 (m, 2H), 4.63 (d, J = 6.1 Hz, 1H), 4.04 (s, 3H), 3.91-3.86 (m, 2H), 3.44-3.34 (m, 2H), 3.18-3.11 (m, 2H), 3.01-2.98 (m, 1H), 1.96-1.93 (m, 1H), 1.77-1.69 (m, 1H), 1.64-1.54 (m, 1H), 1.45-1.36 (m, 1H).
LC-MS (method 1): t_{R} = 1.61 min; [M+1]⁺ = 456.

### Example 6: (S)-1-((2S,5R)-5-aminotetrahydro-2H-pyran-2-yl)-2-(3-fluoro-6-methoxy-1,5-naphthyridin-4-yl)ethanol:

### Variant 1:

A 2.5 L sulfier flask was charged with the compound of Example 4 (200 g, 1 eq.) and iPrOH (400 mL). The suspension was heated to 70°C and 5*M* HCl in iPrOH (900 mL, 10 eq.) was added over a period of 40 min at 60 to 65°C. The resulting solution was stirred at 63°C for 30 min. The product precipitated during that time. The suspension was cooled to 7°C, filtered and washed with TBME (500 mL) to yield a grey solid. A 4 L double jacket flask was charged with the HCl salt (about 200 g), DCM (1600 mL) and 2*N* NaOH (600 mL). The mixture was stirred for 15 min until two clear layers were obtained. The aq. layer was discarded and the org. layer was washed with water (400 mL). The org. layer was concentrated at 80°C external temperature and 1350 mL solvent were removed. Hept (3 L) was added at 40°C and distillation was continued. 250 mL solvent were removed. The suspension was cooled to 20°C and filtered. The product was dried on a rotary evaporator at 50°C to yield an off-white solid (117 g; 77% yield).
¹H-NMR (d6-DMSO): δ = 8.75 (s, 1H), 8.28 (d, J = 9.0 Hz, 1H), 7.23 (d, J = 9.0 Hz, 1H), 4.59 (d, J = 5.9 Hz, 1H), 4.03 (s, 3H), 3.93 (m, 1H), 3.80 (dd, J = 10.5, 4.0 Hz, 1H), 3.34 (s, 1H), 3.13 (m, 2H), 2.84 (t, J = 10.5 Hz, 1H), 2.59 (m, 1H), 1.92 (m, 1H), 1.65 (m, 1H), 1.53 (m, 1H), 1.35 (m, 2H), 1.14 (m, 1H).
LC-MS (method 1), t_{R} = 0.95 min; [M+1]⁺ = 322.

### Variant 2:

General Procedure A was performed on the compound of Example 3, however stirring at - 78°C for 2 h (instead of 1 h). The resulting yellow solution was warmed up to RT and carefully quenched by the addition of aq. 32% NaOH. The mixture was stirred overnight at RT and extracted with DCM twice. The combined org. extracts were concentrated under vacuum (50°C) to afford the title compound as a crude white solid (60 mg; 78% yield) consisting of a 86 / 14 mixture of diastereoisomers (determined by chiral HPLC method 2). The product has NMR data equivalent to those obtained for the product of Variant 1.

### Variant 3:

General Procedure A was performed on 1-((*2S,-5R*)-*5*-aminotetrahydro-2*H*-pyran-2-yl)-2-(3-fluoro-6-methoxy-1,5-naphthyridin-4-yl)ethanone hydrochloride, however stirring at - 78°C for 6 h (instead of 1 h) and using 9.5 eq. (1.83 mL, 1.83 mmol) of DIBAH (instead of 4.5 eq.). The resulting yellow solution was warmed up to RT and carefully quenched by the addition of water. The mixture was basified with 32% aq. NaOH and extracted twice with DCM. The combined org. extracts were concentrated under vacuum (50°C) to afford the title compound (100 mg; yield) as a crude yellow solid consisting of a 86:14 mixture of diastereoisomers (determined by chiral HPLC as described above).

The product has NMR data equivalent to those obtained for the product of Variant 1.

## Claims

1. Use of the compound of formula I-1_{BOC} 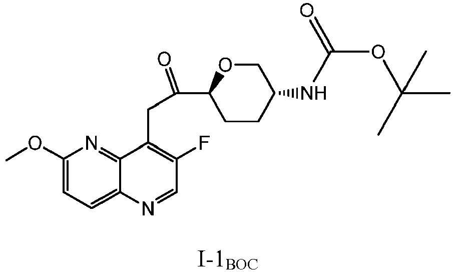 in a process for the stereoselective preparation of the compound of formula I-4_{BOC} 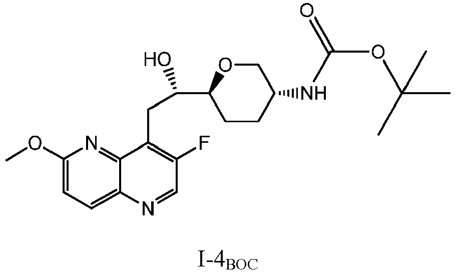 said process comprising the reaction, in a polar aprotic solvent or a polar aprotic solvent mixture, of said compound of formula I-1_{BOC}
a) either with DIBAH,
b) or with triisopropoxyaluminium, dimethylaluminium chloride or diethylaluminium chloride in the presence of iPrOH.

2. Use according to claim 1, wherein the process comprises the reaction, in a polar aprotic solvent or a polar aprotic solvent mixture, of the compound of formula I-1_{BOC} with DIBAH.

3. Use according to claim 2, wherein the polar aprotic solvent of the process comprises THF.

4. Use according to claim 2 or 3, wherein the reaction with DIBAH is performed using from 2 to 4 equivalents of DIBAH per equivalent of compound of formula I-1_{BOC}.

5. Use according to claim 1, wherein the process comprises the reaction, in a polar aprotic solvent or a polar aprotic solvent mixture, of the compound of formula I-1_{BOC} with triisopropoxyaluminium, dimethylaluminium chloride or diethylaluminium chloride in the presence of iPrOH.

6. Use of the compound of formula I-1_{BOC} as defined in claim 1 in a process for the stereoselective preparation of the compound of formula I-5 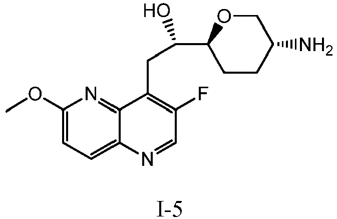 said process comprising:
a) the reaction of the compound of formula I-1_{BOC}, in a polar aprotic solvent or solvent mixture, either with DIBAH, or with triisopropoxyaluminium or dimethylaluminium chloride in the presence of iPrOH, affording the compound of formula I-4_{BOC} 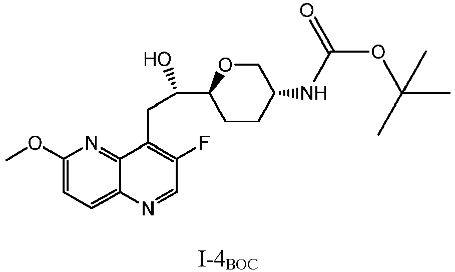 and
b) the deprotection reaction, in acidic conditions, of the compound of formula I-4_{BOC}, affording the compound of formula I-5.

7. A process for the stereoselective preparation of a compound of formula I-4 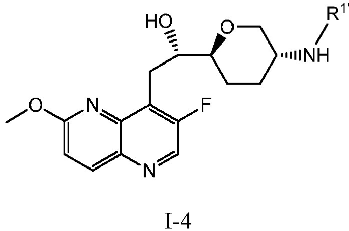 wherein R^{1'} represents hydrogen or a group -CO-R^{2'} wherein R² represents *tert*-butoxy, benzyloxy or 4-methoxybenzyloxy, said process comprising the reaction, in a polar aprotic solvent or a polar aprotic mixture of solvents, of a compound of formula I-1 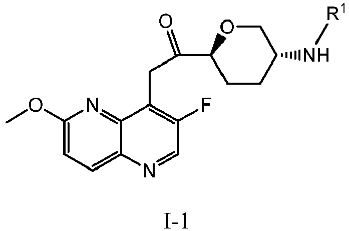 wherein R¹ represents hydrogen or a group -CO-R² wherein R² represents trifluoromethyl, *tert*-butoxy, benzyloxy or 4-methoxybenzyloxy
a) either with DIBAH,
b) or with triisopropoxyaluminium, dimethylaluminium chloride or diethylaluminium chloride in the presence of iPrOH.

8. A process according to claim 7, which comprises the reaction of the compound of formula I-1 with DIBAH.

9. A process according to claim 7, which comprises the reaction of the compound of formula I-1 with triisopropoxyaluminium, dimethylaluminium chloride or diethylaluminium chloride in the presence of iPrOH.

## Patentansprüche

1. Verwendung der Verbindung von Formel I-1_{BOC}: 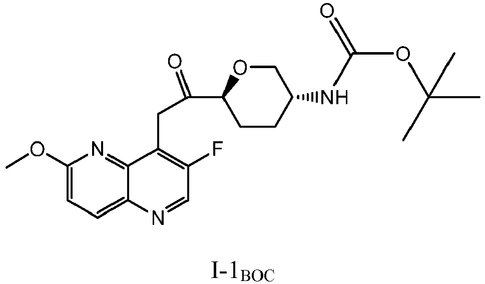 in einem Verfahren zur stereoselektiven Herstellung der Verbindung von Formel I-4_{BOC}: 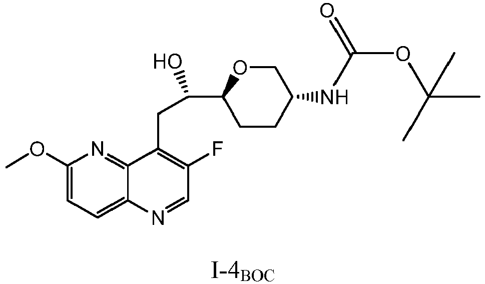 wobei das genannte Verfahren die Reaktion, in einem polaren aprotischen Lösungsmittel oder einem polaren aprotischen Lösungsmittelgemisch, der genannten Verbindung von Formel I-1_{BOC}
a) entweder mit DIBAH,
b) oder mit Triisopropoxyaluminium, Dimethylaluminiumchlorid oder Diethylaluminiumchlorid in Anwesenheit von iPrOH beinhaltet.

2. Verwendung nach Anspruch 1, wobei das Verfahren die Reaktion, in einem polaren aprotischen Lösungsmittel oder einem polaren aprotischen Lösungsmittelgemisch, der Verbindung von Formel I-1_{BOC} mit DIBAH beinhaltet.

3. Verwendung nach Anspruch 2, wobei das polare aprotische Lösungsmittel des Verfahrens THF beinhaltet.

4. Verwendung nach Anspruch 2 oder 3, wobei die Reaktion mit DIBAH unter Verwendung von 2 bis 4 Äquivalenten von DIBAH pro Äquivalent der Verbindung von Formel I-1_{BOC} durchgeführt wird.

5. Verwendung nach Anspruch 1, wobei das Verfahren die Reaktion, in einem polaren aprotischen Lösungsmittel oder einem polaren aprotischen Lösungsmittelgemisch, der Verbindung von Formel I-1_{BOC} mit Triisopropoxyaluminium, Dimethylaluminiumchlorid oder Diethylaluminiumchlorid in Anwesenheit von iPrOH beinhaltet.

6. Verwendung der Verbindung von Formel I-1_{BOC} nach Anspruch 1 in einem Verfahren zur stereoselektiven Herstellung der Verbindung von Formel I-5: 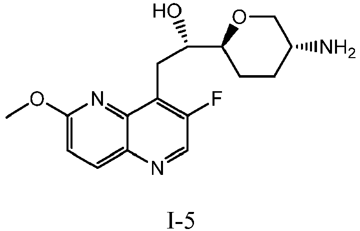 wobei das genannte Verfahren Folgendes beinhaltet:
a) die Reaktion der Verbindung von Formel I-1_{Boc}, in einem polaren aprotischen Lösungsmittel oder Lösungsmittelgemisch, entweder mit DIBAH oder mit Triisopropoxyaluminium oder Dimethylaluminiumchlorid in Anwesenheit von iPrOH, so dass die Verbindung von Formel I-4_{BOC} erhalten wird: 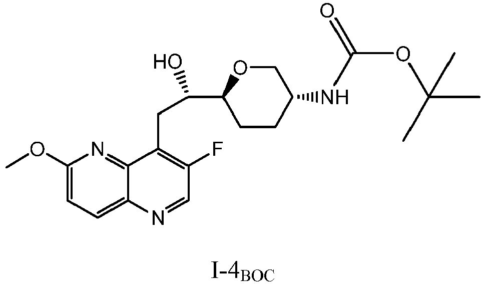 und
b) die Entschützungsreaktion, unter sauren Bedingungen, der Verbindung von Formel I-4_{BOC}, so dass die Verbindung von Formel I-5 erhalten wird.

7. Verfahren zur stereoselektiven Herstellung einer Verbindung von Formel I-4: 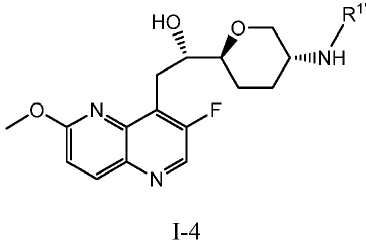 wobei R^{1'} Wasserstoff oder eine Gruppe -CO-R^{2'} repräsentiert, wobei R^{2'} *tert*-Butoxy, Benzyloxy oder 4-Methoxybenzyloxy repräsentiert, wobei das genannte Verfahren die Reaktion, in einem polaren aprotischen Lösungsmittel oder einem polaren aprotischen Lösungsmittelgemisch, einer Verbindung von Formel I-1: 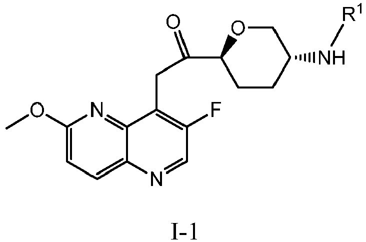 wobei R¹ Wasserstoff oder eine Gruppe -CO-R² repräsentiert, wobei R² Trifluormethyl, *tert*-Butoxy, Benzyloxy oder 4-Methoxybenzyloxy repräsentiert,
a) entweder mit DIBAH,
b) oder mit Triisopropoxyaluminium, Dimethylaluminiumchlorid oder Diethylaluminiumchlorid in Anwesenheit von iPrOH beinhaltet.

8. Verfahren nach Anspruch 7, das die Reaktion der Verbindung von Formel I-1 mit DIBAH beinhaltet.

9. Verfahren nach Anspruch 7, das die Reaktion der Verbindung von Formel I-1 mit Triisopropoxyaluminium, Dimethylaluminiumchlorid oder Diethylaluminiumchlorid in Anwesenheit von iPrOH beinhaltet.

## Revendications

1. Utilisation du composé de formule I-1_{BOC} 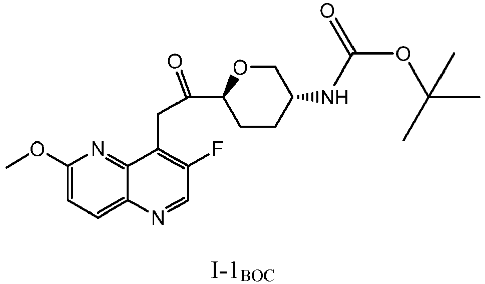 dans un procédé de préparation stéréosélective du composé de formule I-4_{BOC} 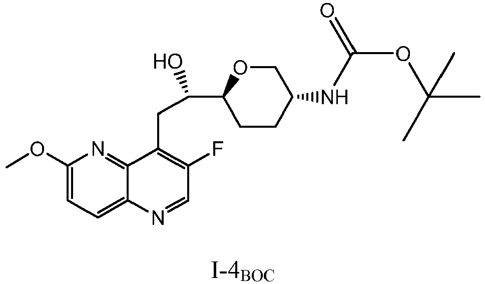 ledit procédé comprenant la réaction, dans un solvant aprotique polaire ou un mélange de solvants aprotique polaire, dudit composé de formule I-1_{BOC}
a) soit avec du DIBAH,
b) soit avec du triisopropoxyaluminium, du chlorure de diméthylaluminium ou du chlorure de diéthylaluminium en la présence d'iPrOH.

2. Utilisation selon la revendication 1, dans laquelle le procédé comprend la réaction, dans un solvant aprotique polaire ou un mélange de solvants aprotique polaire, du composé de formule I-1_{BOC} avec du DIBAH.

3. Utilisation selon la revendication 2, dans laquelle le solvant aprotique polaire du procédé comprend du THF.

4. Utilisation selon la revendication 2 ou 3, dans laquelle la réaction avec le DIBAH est effectuée en employant de 2 à 4 équivalents de DIBAH par équivalent du composé de formule I-1_{BOC}.

5. Utilisation selon la revendication 1, dans laquelle le procédé comprend la réaction, dans un solvant aprotique polaire ou un mélange solvant aprotique polaire, du composé de formule I-1_{BOC} avec du triisopropoxyaluminium, du chlorure de diméthylaluminium ou du chlorure de diéthylaluminium en la présence d'iPrOH.

6. Utilisation du composé de formule I-1_{BOC} tel que défini à la revendication 1 dans un procédé de préparation stéréosélective du composé de formule I-5 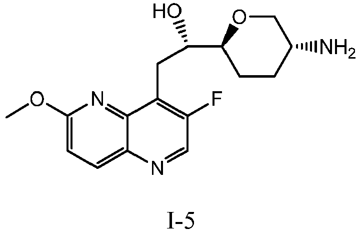 ledit procédé comprenant :
a) la réaction du composé de formule I-1_{BOC}, dans un solvant ou un mélange de solvants aprotique polaire, avec du DIBAH ou avec du triisopropoxyaluminium ou du chlorure de diméthylaluminium en la présence d'iPrOH, donnant le composé de formule I-4_{BOC} 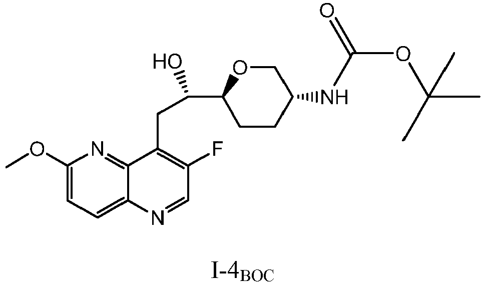 et
b) la réaction de déprotection, dans des conditions acides, du composé de formule I-4_{BOC}, donnant le composé de formule I-5.

7. Procédé de préparation stéréosélective d'un composé de formule I-4 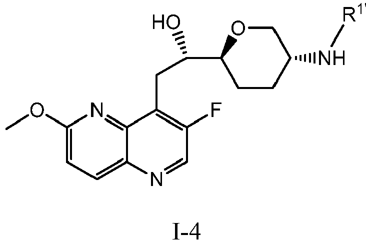 dans lequel R¹ représente un hydrogène ou un groupement -CO-R^{2'}, où R^{2'} représente un *tert*-butoxy, un benzyloxy ou un 4-méthoxybenzyloxy, ledit procédé comprenant la réaction, dans un solvant aprotique polaire ou un mélange de solvants aprotique polaire, d'un composé de formule I-1 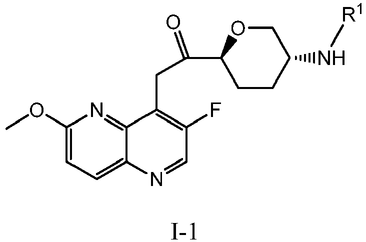 dans lequel R¹ représente un hydrogène ou un groupement -CO-R², où R² représente un trifluorométhyle, un *tert*-butoxy, un benzyloxy ou un 4-méthoxybenzyloxy,
a) soit avec du DIBAH,
b) soit avec du triisopropoxyaluminium, du chlorure de diméthylaluminium ou du chlorure de diéthylaluminium en la présence d'iPrOH.

8. Procédé selon la revendication 7, qui comprend la réaction du composé de formule I-1 avec du DIBAH.

9. Procédé selon la revendication 7, qui comprend la réaction du composé de formule I-1 avec du triisopropoxyaluminium, du chlorure de diméthylaluminium ou du chlorure de diéthylaluminium en la présence d'iPrOH.
